(19) Europäisches Patentamt
European Patent Office
Office européen des brevets

(11) Veröffentlichungsnummer: **0 384 313 A1**

(12) # EUROPÄISCHE PATENTANMELDUNG

(21) Anmeldenummer: **90103019.7**

(22) Anmeldetag: **16.02.90**

(51) Int. Cl.[5]: **C07D 215/52, C07D 405/04, C07D 491/056, B41M 5/136, //(C07D491/056,317:00,215:00)**

(30) Priorität: **22.02.89 DE 3905339**

(43) Veröffentlichungstag der Anmeldung: **29.08.90 Patentblatt 90/35**

(84) Benannte Vertragsstaaten: **CH DE FR GB IT LI**

(71) Anmelder: **BASF Aktiengesellschaft
Carl-Bosch-Strasse 38
D-6700 Ludwigshafen(DE)**

(72) Erfinder: **Raulfs, Friedrich-Wilhelm, Dr.
Torwiesenstrasse 1
D-6800 Mannheim 1(DE)**
Erfinder: **Mayer, Udo, Dr.
Max-Slevogt-Strasse 27
D-6710 Frankenthal(DE)**

(54) **Chinolin-4-carbonsäurederivate und deren Verwendung.**

(57) Es werden Chinolin-4-carbonsäurederivate der allgemeinen Formel I

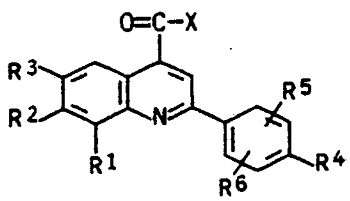

(I)

in der einer der Reste

$R^1$, $R^2$ und $R^3$ für Wasserstoff, die beiden anderen unabhängig voneinander für Wasserstoff, Fluor, Chlor, Brom, lineares und verzweigtes $C_1$-$C_5$ Alkyl, Hydroxy, $C_1$-$C_{10}$ Alkoxy, das im Alkyl durch 1 oder 2 Sauerstoffatome unterbrochen sein kann oder für

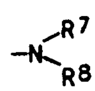

stehen, wobei

$R^7$ Wasserstoff, Methyl oder Ethyl und

$R^8$ $C_1$-$C_4$ Alkanoyl, Benzoyl, p-Chlorbenzoyl, $C_1$-$C_6$ Alkyl, Benzyl oder p-Chlorbenzyl ist, oder

$R^1$ und $R^2$ zusammen einen ankondensierten Benzolring,

$R^2$ und $R^3$ zusammen gegebenenfalls durch $C_1$- bis $C_4$-Alkyl substituierte Methylendioxy oder Ethylendioxy,

$R^4$ Hydroxy, $C_1$-$C_{10}$ Alkoxy, das durch 1 bis 4 Sauerstoffatome unterbrochen sein kann, oder gegebenenfalls durch Chlor substituiertes Phenyl-$C_1$-$C_2$-alkoxy,

$R^5$ Hydroxy oder $C_1$-$C_{10}$ Alkoxy, wobei orthoständiges $R^4$ und $R^5$ zusammen auch Methylendioxy oder 1,2-Ethylendioxy sein können,

$R^6$ Wasserstoff, Hydroxy, Methoxy, Ethoxy, Chlor, Fluor oder $C_1$-$C_4$ Alkyl und

EP 0 384 313 A1

X $C_1$-$C_{20}$ Alkoxy, Benzyloxy, p-Chlorbenzyloxy, Phenylethyloxy, Cyclopentoxy oder Cyclohexoxy oder

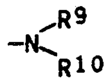

bedeuten, wobei
$R^9$ Wasserstoff, Methyl oder Ethyl und
$R^{10}$ Wasserstoff, $C_1$-$C_{20}$ Alkyl, gegebenenfalls ein- oder zweimal durch Chlor, Methoxy oder Methyl substituiertes Phenyl oder ein Rest der Formel

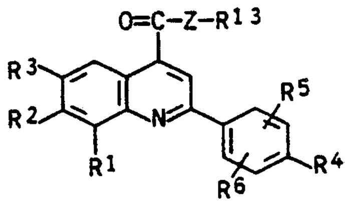

(II) ,

sind, in dem
$R^1$ bis $R^6$ die oben angegebene Bedeutung haben und
Z Sauerstoff oder >N-$R^9$ und
$R^{13}$ lineares oder verzweigtes $C_2$-$C_{10}$-Alkylen, das durch bis zu 4 Sauerstoffatome unterbrochen sein kann, oder 1,2-, 1,3- oder 1,4-Xylylen bedeuten.
Die Verbindungen I sind Farbbildner für druck- und thermoempfindliche Aufzeichnungsmaterialien.

## Chinolin-4-carbonsäurederivate und deren Verwendung

Aus dem Stand der Technik sind Aufzeichnungsmaterialien mit Farbbildnern, die gelbe bis orange Färbungen liefern, bekannt. So werden in der DE-OS 2 227 597 Pyridinverbindungen beschrieben, die auf mit Elektronenacceptoren beschichtetem Papier gelbe bis orange Färbungen geben. Aus den EP-A-109 930 und EP-A-159 295 sind Bischinazolinverbindungen bekannt, die als Farbbildner für druck- oder wärmeempfindliche Aufzeichnungsmaterialien geeignet sind. Weiterhin sind aus der EP-A-256 180 und GB-A-2136823 Farbbildner auf Basis Dihydroxystyrylchinolin bekannt.

Die Farbbildner des Standes der Technik haben den Nachteil, daß sie sowohl im neutralen als auch im protonierten Zustand nur eine geringe Lichtechtheit haben.

Aufgabe der vorliegenden Anmeldung war es, hinsichtlich der Lichtechtheit verbesserte Farbbildner bereitzustellen, die gelbe bis orange Färbungen liefern.

Diese Aufgabe wird mit Hilfe der erfindungsgemäßen Chinolin-4-carbonsäurederivate gelöst.

Die Erfindung betrifft Chinolincarbonsäurederivate der Formel I

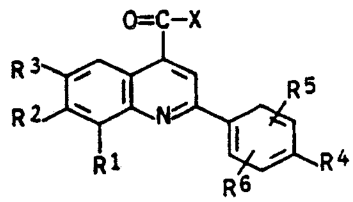

(I)

in der einer der Reste

$R^1$, $R^2$ und $R^3$ für Wasserstoff, die beiden anderen unabhängig voneinander für Wasserstoff, Fluor, Chlor, Brom, lineares und verzweigtes $C_1$-$C_5$ Alkyl, Hydroxy, $C_1$-$C_{10}$ Alkoxy, das im Alkyl durch 1 oder 2 Sauerstoffatome unterbrochen sein kann oder für

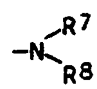

stehen, wobei

$R^7$ Wasserstoff, Methyl oder Ethyl und

$R^8$ $C_1$-$C_4$ Alkanoyl, Benzoyl, p-Chlorbenzoyl, $C_1$-$C_6$ Alkyl, Benzyl oder p-Chlorbenzyl ist, oder

$R^1$ und $R^2$ zusammen einen ankondensierten Benzolring,

$R^2$ und $R^3$ zusammen gegebenenfalls durch $C_1$- bis $C_4$-Alkyl substituierte Methylendioxy oder Ethylendioxy,

$R^4$ Hydroxy, $C_1$-$C_{10}$ Alkoxy, das durch 1 bis 4 Sauerstoffatome unterbrochen sein kann, oder gegebenenfalls durch Chlor substituiertes Phenyl-$C_1$-$C_2$-alkoxy,

$R^5$ Hydroxy oder $C_1$-$C_{10}$ Alkoxy, wobei orthoständiges $R^4$ und $R^5$ zusammen auch Methylendioxy oder 1,2-Ethylendioxy sein können,

$R^6$ Wasserstoff, Hydroxy, Methoxy, Ethoxy, Chlor, Fluor oder $C_1$-$C_4$ Alkyl und

X $C_1$-$C_{20}$ Alkoxy, Benzyloxy, p-Chlorbenzyloxy, Phenylethyloxy, Cyclopentoxy oder Cyclohexoxy oder

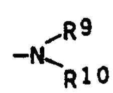

bedeuten, wobei

$R^9$ Wasserstoff, Methyl oder Ethyl und

$R^{10}$ Wasserstoff, $C_1$-$C_{20}$ Alkyl, gegebenenfalls ein- oder zweimal durch Chlor, Methoxy oder Methyl substituiertes Phenyl oder ein Rest der Formel

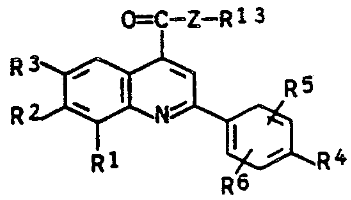

(II) ,

sind, in dem
$R^1$ bis $R^6$ die oben angegebene Bedeutung haben und
Z Sauerstoff oder >N-$R^9$ und
$R^{13}$ lineares oder verzweigtes $C_2$-$C_{10}$-Alkylen, das durch bis zu 4 Sauerstoffatome unterbrochen sein kann, oder 1,2-, 1,3- oder 1,4-Xylylen bedeuten.

Die Chinolin-4-carbonsäurederivate der Erfindung geben mit Elektronenacceptoren, wie sie in gewöhnlichen CF-Schichten verwendet werden, intensive gelbe bis orange Färbungen, die im Vergleich zu Färbungen, die mit den in EP-A-109 930, GB-A-2 136 823 und DE-OS-22 27 597 beschriebenen Farbbildnern erhalten werden, in der Lichtechtheit überlegen sind.

Neben Wasserstoff, Fluor, Chlor, Brom und Hydroxy sind für $R^1$, $R^2$ und $R^3$ zu nennen:

a) lineares oder verzweigtes $C_1$-$C_5$ Alkyl, z.B. Methyl, Ethyl, Propyl, Isopropyl, Butyl, Isobutyl, tert. Butyl, Pentyl und Isoamyl;

b) $C_1$-$C_{10}$ Alkoxy, das im Alkyl durch 1 oder 2 Sauerstoffatome unterbrochen sein kann, wie Methoxy, Ethoxy, Propoxy, Isopropoxy, Butoxy, Isobutoxy, tert. Butoxy, Pentoxy, Hexoxy, 2-Ethylhexoxy und 2-(Ethoxy)ethoxy;

c) ein Rest der Formel

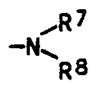

z.B. Acetylamino, Propionylamino, Benzoylamino, Acetylmethylamino, N,N-Dimethylamino, N,N-Diethylamino, N-Methyl-N-butylamino, oder N-Ethyl-N-hexylamino. Einer der Reste $R^1$, $R^2$, $R^3$ muß Wasserstoff sein.

$R^1$ und $R^2$ können auch zusammen für einen ankondensierten Benzolring stehen.

Vorzugsweise stehen $R^1$ und $R^3$ für Wasserstoff und $R^2$ für Wasserstoff, Methyl oder Methoxy.

Ferner sind für $R^2$ und $R^3$ zusammen gegebenenfalls durch Methyl substituiertes Methylen- oder Ethylendioxy bevorzugt.

Neben Hydroxy steht $R^4$ für $C_1$-$C_{10}$ Alkoxy, das durch 1, 2, 3 oder 4 Sauerstoffatome unterbrochen sein kann oder für gegebenenfalls durch Chlor substituiertes Phenyl-$C_1$-$C_2$-alkoxy. Im einzelnen sind z.B. zu nennen: Methoxy, Ethoxy, Propoxy, Isopropoxy, Butoxy, Isobutoxy, tert. Butoxy, Pentoxy, Hexoxy, 2-Ethylhexoxy, Octyloxy, Decyloxy, 2-(Ethoxy)ethoxy, 2-(Propoxy)ethoxy und 2′-(Ethoxy-2-ethoxyethylenoxy; Benzyloxy, p-Chlorbenzyloxy und Phenylethyloxy.

$R^5$ steht außer für Hydroxy für $C_1$-$C_{10}$-Alkoxy wie Methoxy, Ethoxy, Propoxy, Isopropoxy, Butoxy, Isobutoxy, tert. Butoxy, Pentoxy, Hexoxy, 2-Ethylhexoxy und Octyloxy.

Für $R^6$ sind neben den bestimmt genannten, z.B. Methyl, Ethyl, Propyl, Isopropyl, Butyl und Isobutyl zu nennen.

Ist $R^4$ zusammen mit $R^5$ eine Methylendioxy- oder 1,2-Ethylendioxy-Gruppe, sind die Reste

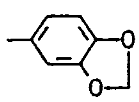

und

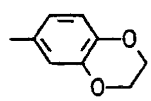

hervorzuheben.

Beispiele bevorzugter Kombinationen, die Substituenten $R^4$, $R^5$ und $R^6$ enthalten, sind in der Tabelle 1 zusammengestellt.

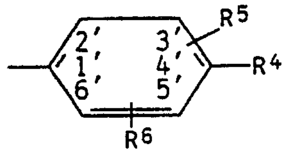

Tabelle 1

| $R^4$ | $R^5$ | $R^6$ |
|---|---|---|
| $-OCH_3$ | 2'-$OCH_3$ | H |
| $-OCH_3$ | 3'-$OCH_3$ | H |
| $-OCH_3$ | 3'-$OCH_3$ | 5'-$OCH_3$ |
| $-OC_2H_5$ | 2'-$OC_2H_5$ | H |
| -OH | 3'-$OCH_3$ | H |
| -OH | 3'-$OCH_3$ | 5'-Cl |
| $-OCH_3$ | 5'-$OCH_3$ | 2'-$CH_3$ |
| $-OCH_3$ | 5'-$OCH_3$ | 2'-$C_2H_5$ |
| $-OCH_3$ | 3'-$OCH_3$ | 2'-$OCH_3$ |
| $-OCH_3$ | 2'-OH | $-OCH_3$ |
| $-OCH_3$ | 3'-$OCH_3$ | 5'-$CH(CH_3)_2$ |
| $-OCH_3$ | 2'-$OCH_3$ | 5'-$OCH_3$ |
| $-OC_2H_5$ | 3'-$OCH_3$ | H |
| $-OC_3H_7$ | 3'-$OCH_3$ | H |
| $-OC_4H_9$ | 3'-$OCH_3$ | H |
| $-OC_4H_9$ | 2'-$OC_4H_9$ | H |

Besonders bevorzugt sind Farbbildner I, bei denen $R^5$ in 2'- oder 3'-Stellung steht und $R^6$ Wasserstoff ist.

Neben den bestimmt genannten Resten steht

X für $C_1$-$C_{20}$ Alkoxy wie Methoxy, Ethoxy, Propoxy, Isopropoxy, Butoxy, Isobutoxy, sec. Butoxy, tert. Butoxy, Pentoxy, Isoamyloxy, tert. Amyloxy, Neopentyloxy, Hexyloxy, 2-Ethylhexyloxy, Heptoxy, Octyloxy, Nonyloxy, Decyloxy, Dodecyloxy, Cetyloxy und Octandecyloxy oder für einen Rest der Formel

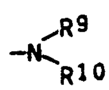

$R^9$ steht für Wasserstoff, Methyl oder Ethyl. Für $R^{10}$ sind neben Wasserstoff zu nennen:

i) $C_1$-$C_{20}$-Alkyl wie Methyl, Ethyl, Propyl, Isopropyl, Butyl, Isobutyl, sec. Butyl, tert. Butyl, Hexyl, 2-Ethylhexyl, Octyl und Dodecyl;

ii) gegebenenfalls substituiertes Phenyl wie Phenyl, 4-Chlorphenyl, 4-Methoxyphenyl, 4-Methylphenyl, 2-Methylphenyl, 3-Methylphenyl, 3,4-Dimethylphenyl und

iii) ein Rest der Formel II.

Ist X ein Rest der Formel II, so kann Z Sauerstoff oder >N-$R^9$ sein. Als Brückenglied $R^{13}$ kommen neben den bestimmt genannten Resten lineares oder verzweigtes $C_2$-$C_{10}$-Alkylen in Betracht, das durch bis zu 4 Sauerstoffatome unterbrochen sein kann. Im einzelnen sind z.B. zu nennen: $-(CH_2)_3-$, $-(CH_2)_3-$, $-CH_2-CH-CH_3$, $-(CH_2)_4-$, $CH_3-CH-CH-CH_3$, $-(CH_2)_5-$, $-(CH_2)_2-C(CH_3)_2$, $-CH_2-C(CH_3)_2-CH_2-$, $(CH_2)_6$, $CH_3-CH-(CH_2)_3-CH-CH_3$, $(CH_3)_2-C-CH_2-CH-CH_3$, $-CH_2-C(C_2H_4)_2-CH_2--CH_2-C(CH_3)(C_3H_7)-CH_2-$, $-(CH_2)-_8$, $-CH_2-C(CH_3)_2-C-CH-(CH_3)_2$, $-CH_2-C(C_2H_5)(C_4H_9)-CH_2-$, $-CH_2-C(CH_3)_2-CH_2-CH(CH_3)-(CH_2)_2-$, $-C(C_4H_7)_2-(CH_2)_2-$, $-(C_2H_4-O)_3-C_2H_4-$ und $C_3H_6-O-C_2H_4-O-C_3H_6-$.

Die erfindungsgemäßen Chinolinverbindungen (I) sind farblose bis schwach gelbliche Verbindungen, deren Lösungen in inerten organischen Lösungsmitteln wie partiell hydrierten Di- oder Terphenyl, Alkylbenzole, Alkylnaphthalinen, alkylierte Dibenzylbenzolen, Paraffinöl oder Mineralöl in Kontakt mit Elektronenacceptoren, wie Carbon-, Sulfon- oder Mineralsäuren, Kaolin, Bentonit, aktivierten sauren Ton, Aluminiumsilikat, Attapulkit oder sauer reagierenden organischen Polymeren, beispielsweise Kondensationsprodukten aus Formaldehyd und Phenolen oder Phenolsulfonsäuren, gelbe bis orange Färbungen liefern. Wegen dieser Eigenschaften können diese Verbindungen in Mikrokapseln eingeschlossen werden. Bei Druck tritt im Kontakt mit Elektronenacceptoren an der Druckstelle Farbbildung ein.

Geeignete Verfahren zur Herstellung von Mikrokapseln sind z.B. aus der US-A-2 800 457, US-A-2 800 458, DE-A-21 19 933 und EP-A-26 914 bekannt. Die erfindungsgemäßen Verbindungen können auch nach dem in der US-A-3 103 404 beschriebenen Verfahren in Wachs- oder Öl-Wachsmischungen fein verteilt werden. Damit beschichtetes Papier gibt auf Druck den Farbstoff frei und auf einem vorderseitig mit Elektronenacceptoren beschichtetem Papier tritt die Farbbildung ein.

Ebenso können die Chinolin-4-carbonsäurederivate z.B. nach DE-A-2 228 581 oder DE-A-21 10 854 als Farbbildner in wärmeempfindlichen Aufzeichnungsmaterialien oder z.B. in Kopiersystemen der GB-A-2 029 591 oder GB-A-2 162 652 verwendet werden.

Die Lichtechtheit der entwickelten Färbungen ist bei den Chinolin-4-carbonsäureverbindungen (I) besser als die, der mit den Verbindungen des oben angegebenen Standes der Technik erhaltenen Färbungen.

Chinolin-4-carbonsäuren, die zur Synthese von Verbindungen der Formel I benötigt werden, können nach der Methode von W. Pfitzinger (J. Prakt. Chem. 38, 582 (1888)) hergestellt werden, nach der ein Isatin der allgemeinen Formel (III)

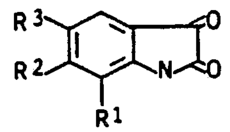

(III)

z.B. in starker alkoholischer Alkalilauge, vorzugsweise Kaliumhydroxyd in Ethanol, mit einem Acetophenon der Formel (IV)

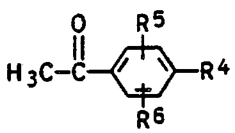

(IV)

umgesetzt wird. Diese Reaktion kann z.B. auch durch Phasentransferkatalyse, analog Dyes and Pigments 8 (1987), Seiten 281-290 durchgeführt werden.

Die erhaltenen Chinolin-4-carbonsäuren werden anschließend nach bekannten Methoden zu (I) verestert oder amidiert.

Eine weitere Methode zur Herstellung von Chinolin-4-carbonsäuren ist in Chem. Ber. 20, S. 277 (1887) beschrieben. Danach wird ein Anilin der Formel (V)

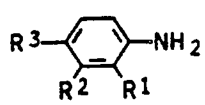

(V)

und ein Aldehyd der Formel VI

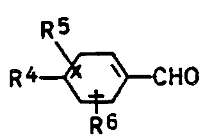

(VI)

mit Brenztraubensäure in einem $C_1$-$C_5$-Alkanol, vorzugsweise in Methanol oder Ethanol bei Rückflußtemperatur umgesetzt. Die entstandenen Chinolincarbonsäuren werden anschließend nach bekannten Methoden zu (I) verestert oder amidiert.

Die folgenden Beispiele sollen die Erfindung näher erläutern. Die Prozentangaben beziehen sich auf das Gewicht.

Beispiel 1

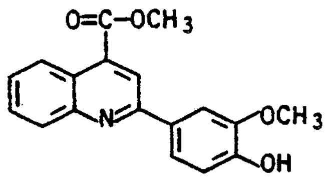

Zu 75,5 g Vanillin und 46 g Anilin wurden in 500 ml Ethanol zwischen 0 bis 5 °C 44 g Brenztraubensäure getropft. Nach zweistündigem Rühren bei Raumtemperatur wurde noch weitere 2 Stunden unter Rückfluß erhitzt. Beim Abkühlen auf Raumtemperatur schieden sich 42,2 g 2-(4′-Hydroxy-3′-methoxyphenyl)-chinolin-4-carbonsäure ab.

14,7 g dieser Carbonsäure wurden in 64 g Methanol aufgenommen. Bei Rückflußtemperatur wurde während 1 1/2 Stunden Chlorwasserstoffgas eingeleitet. Nach dem Abkühlen wurde mit konzentrierter Ammoniaklösung ein pH-Wert von 8,5 eingestellt, die abgeschiedenen Kristalle abgesaugt und aus Methanol umkristallisiert. Es wurden 9,8 g 2-(4′-Hydroxy-3′-methoxyphenyl)-chinolin-4-carbonsäuremethylester mit einem Schmelzpunkt von 144 °C erhalten. In Toluol hat die Verbindung ein $\lambda_{max}$ bei 363 nm und in 2 %iger salzsaurer Methanollösung ein $\lambda_{max}$ bei 420 nm.

Analog Beispiel 1 wurden die in der folgenden Tabelle angegebenen Verbindungen hergestellt.

Tabelle 1

| Beispiel Nr. | Verbindung | $\lambda_{max}$ [nm] neutral | sauer | Schmelzpunkt [°C] |
|---|---|---|---|---|
| 2 | 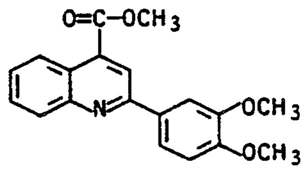 | 366 | 412 | 105 - 109 |
| 3 | 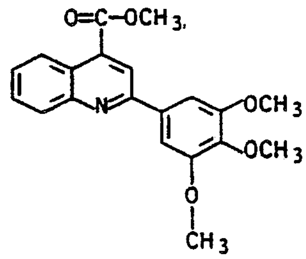 | 359 | 392 | 93 - 96 |
| 4 | 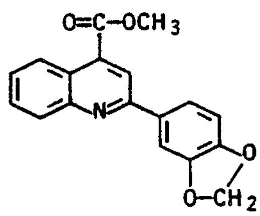 | 363 | 408 | 133 - 136 |
| 5 | 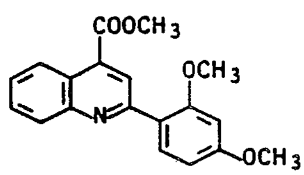 | 356 | 408 | 126 - 128 |
| 6 | 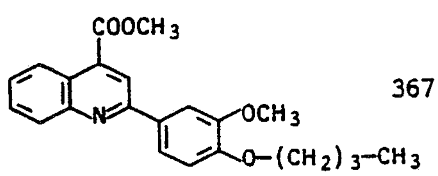 | 367 | 416 | 115,5 |

Beispiel 7

Zu 82,5 g Veratrumaldehyd und 46 g Anilin in 500 ml Ethanol wurden unter Eiskühlung 44 g Brenztraubensäure getropft. Danach wurde 2 Stunden bei Raumtemperatur und anschließend 4 Stunden unter Rückfluß erhitzt. Nach dem Erkalten wurden 48,7 g 2-(3′,4′-Dimethoxyphenyl)-chinolin-4-carbonsäure isoliert.

Beispiel 8

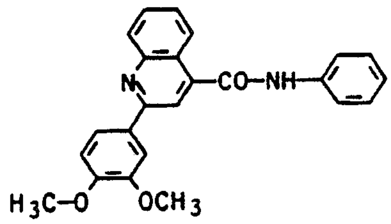

7,72 g, der nach Beispiel 7 hergestellten 2-(3′,4′-Dimethoxyphenyl)-chinolin-4-carbonsäure, wurden bei Raumtemperatur in 40 g Thionylchlorid eingetragen. Es wurde solange gerührt bis sich eine dunkelrote Lösung gebildet hatte. Danach wurde das überschüssige Thionylchlorid im Vakuum bei 40 °C Badtemperatur abdestilliert. Der verbleibende Rückstand wurde bei 25 bis 30 °C in 60 g Anilin eingetragen und kurz auf 80 °C erwärmt. Nach dem Erkalten wurde mit 250 ml Methanol verdünnt, die Kristalle abfiltriert, mit Wasser gewaschen und getrocknet. Ausbeute: 8,72 g 2-(3′,4′-Dimethoxyphenyl)-chinolin-4-carbonsäureanilid, Schmelzpunkt: 235 bis 237 °C, $\lambda_{max}$ in Toluol 354 nm und in 2 %iger methanolischer Salzsäure 398 nm.

Analog Beispiel 8 wurden die Verbindungen der Beispiele 9 bis 12 hergestellt. Wobei im Fall der Beispiele 11 und 12 die entsprechenden Alkohole verwendet wurden. In diesem Falle wurde nach Beendigung der Veresterung das Gemisch mit konzentrierter Ammoniaklösung leicht alkalisch gestellt.

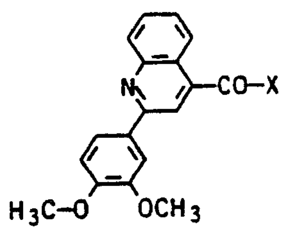

| Beispiel Nr. | X | $\lambda_{max}$ [nm] neutral | sauer | Schmelzpunkt [°C] |
|---|---|---|---|---|
| 9 | 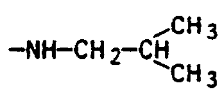 | 350 | 398 | 180,5 |
| 10 | 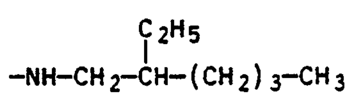 | 351 | 398 | 145 - 147 |
| 11 | 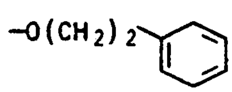 | 366 | 412 | 103 - 106 |
| 12 | $-O(CH_2)_3-CH_3$ | 365 | 411 | 84 - 86 |

Beispiel 13

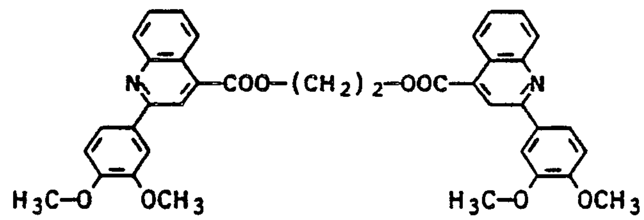

15,44 g der 2-(3′,4′-Dimethoxyphenyl)-chinolincarbonsäure aus Beispiel 7 wurden in 80 g Thionylchlorid gegeben. Nach Beendigung der Reaktion wurde das überschüssige Thionylchlorid im Vakuum bei 40 °C Badtemperatur abdestilliert. Die Hälfte des Säurechlorids wurde portionsweise in 40 g Ethylenglykol eingetragen. Es wurde 1 Stunde auf 70 °C erwärmt. Das Reaktionsgemisch wurde auf 200 ml Wasser gegeben und die Suspension mit Ammoniakwasser leicht alkalisch gestellt. Der entstandene Ester wurde mit Toluol extrahiert. Die abgetrennte organische Phase wurde durch Abdestillieren des Toluol-Wasser-Azeotrops getrocknet. Zu der getrockneten organischen Phase wurden 4 g Pyridin und die andere Hälfte des Säurechlorids hinzugefügt und das Gemisch 1 Stunde auf 75 °C erwärmt. Das entstandene Salz des Diesters wurde abgesaugt, mit Toluol gewaschen und getrocknet. Das Salz wurde in verdünnte Ammoniaklösung eingetragen und gerührt. Nach dem Filtrieren, Waschen und Trocknen wurden 8,8 g Bis(2-(3′,4′-Dimethoxyphenyl)-chinolin-4-carbonsäure)-glykolester erhalten. Schmp. 182 bis 184 °C, $\lambda_{max}$ in Toluol 368 nm; $\lambda_{max}$ in 2 %iger methanolischer Salzsäure: 416 nm.

Die Verbindungen der Beispiele 14 bis 21 wurden analog Beispiel 13 hergestellt.

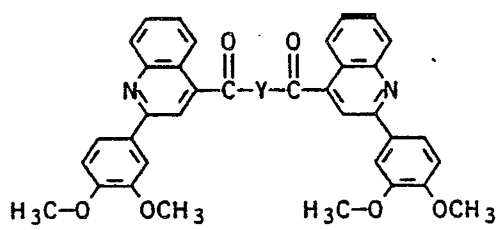

| Beispiel Nr. | Y | $\lambda_{max}$ [nm] neutral | sauer | Schmelzpunkt [°C] |
|---|---|---|---|---|
| 14 | $-O(CH_2)_6O-$ | 366 | 413 | 150 |
| 15 | 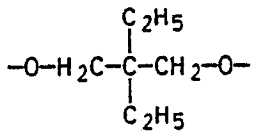 | 368 | 417 | 141 |
| 16 | $-O(CH_2)_4O-$ | 367 | 414 | 151 - 156 |
| 17 | $-O(CH_2)_3O-$ | 368 | 416 | |
| 18 | $-(O(CH_2)_2)_4O-$ | 366 | 413 | 105 - 109 |
| 19 | 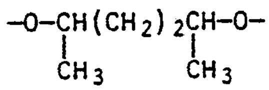 | 366 | 413 | 166 - 171 |
| 20 | 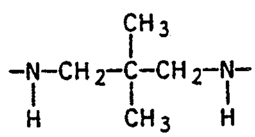 | 354 | 399 | 220 - 223 |
| 21 | 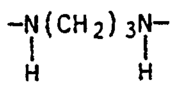 | 355 | 400 | 191 - 196 |

Die als Ausgangsverbindung benötigten 2-(3′,4′-Dimethoxyphenyl)-chinolin-4-carbonsäuren wurden analog Beispiel 7 hergestellt. Die Ethylester wurden aus den Säurechloriden hergestellt. Hierzu wurde die jeweilige Säure mit überschüssigem Thionylchlorid umgesetzt. Das überschüssige Thionylchlorid wurde im Vakuum abdestilliert und der Rückstand unter Kühlung tropfenweise mit Ethanol versetzt. Die Hydrochloride der jeweiligen Chinolin-4-carbonsäureethylester wurden mit konzentrierter Ammoniaklösung leicht alkalisch gestellt und die 2-(3′,4′-Dimethoxyphenyl)-chinolin-4-carbonsäureethylester isoliert.

| Beispiel Nr. | Chinolin-4-carbonsäureester | λmax [nm] neutral | sauer | Schmelzpunkt [°C] |
|---|---|---|---|---|
| 22 | 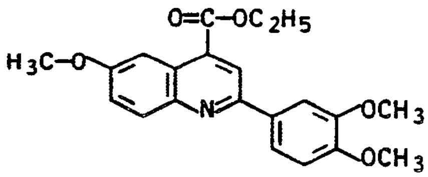 | 383 | 425 | 105 |
| 23 | 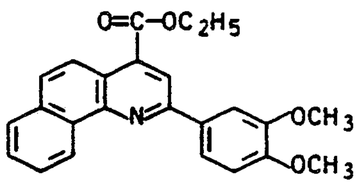 | 384 | 427 | 106 - 110 |
| 24 | 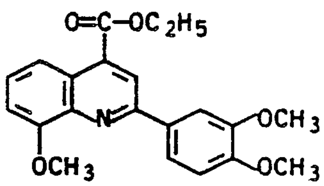 | 364 | 405 | 128 - 131 |
| 25 | 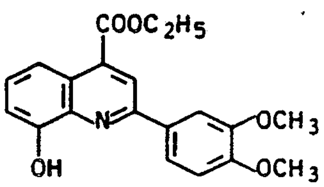 | 368 | 404 | 88 |

| Beispiel Nr. | Chinolin-4-carbonsäureester | $\lambda_{max}$ [nm] neutral | sauer | Schmelzpunkt [°C] |
|---|---|---|---|---|
| 26 | 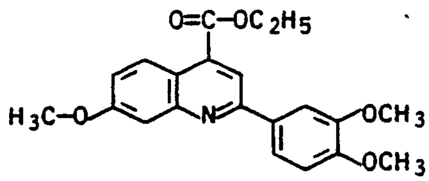 | 371 | 419 | 114 |
| 27 | 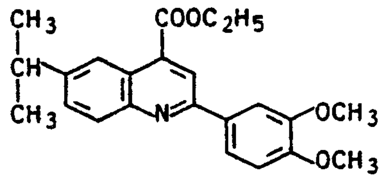 | 369 | 412 | 101 |
| 28 | 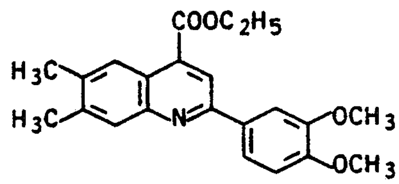 | 368 | 410 | 110 - 112 |
| 29 | 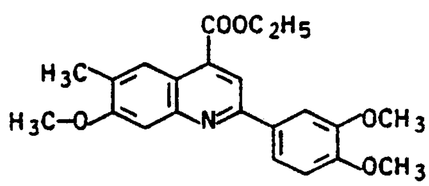 | 371 | 417 | 136 - 138 |
| 30 | 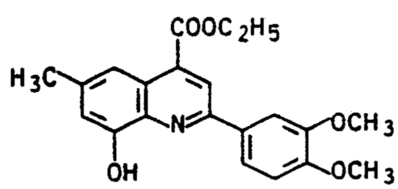 | 361 | 407 | 144 - 150 |
| 31 | 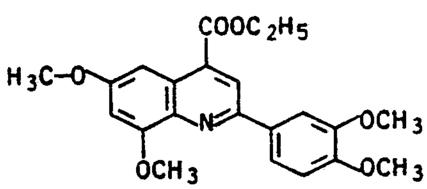 | 391 | 420 | 127 - 129 |

Anilin wurde mit den Aldehyden ACHO in Ethanol gelöst und in der in Beispiel 7 angegebenen Weise zu den entsprechenden Chinolin-4-carbonsäuren umgesetzt. Die Veresterung erfolgte in den in der Tabelle angegebenen Alkoholen H-X bei 80 °C unter Einleiten von Chlorwasserstoffgas. Nach der Neutralisation mit konzentrierter Ammoniaklösung und der üblichen Aufarbeitung wurden die in den der folgenden Tabelle

angegebenen Chinolin-4-carbsonäureester erhalten. Die Bedeutung von A und H-X ist in der Tabelle angegeben.

| Beisp. Nr. | A | | Chinolin-4-carbonsäure-ester | neutral | sauer | Schmelzpunkt [°C] |
|---|---|---|---|---|---|---|
| 32 | 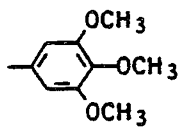 | Phenyl-ethanol | 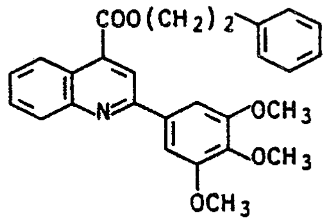 | 359 | 393 | 98 - 101 |
| 33 | 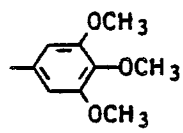 | Butanol | 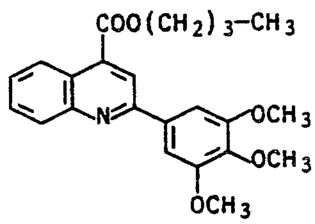 | 358 | 393 | 84 - 86 |
| 34 | 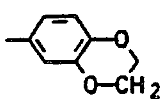 | Butanol | 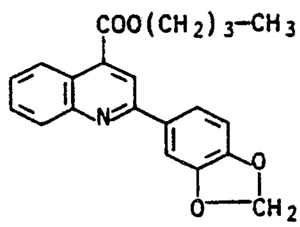 | 362 | 407 | 75 |
| 35 | 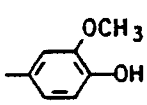 | Butanol | 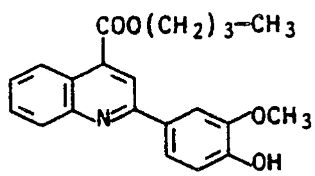 | 363 | 418 | 84 |

| Beisp. Nr. | A | H-X | Chinolin-4-carbonsäure-ester | $\lambda_{max}$ [nm] neutral | sauer | Schmelzpunkt [°C] |
|---|---|---|---|---|---|---|
| 36 | 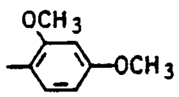 | Butanol | 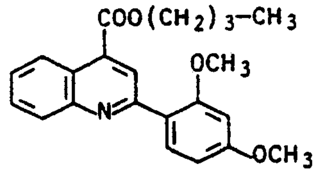 | 356 | 407 | 45 |
| 37 | 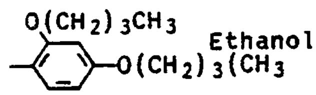 | Ethanol | 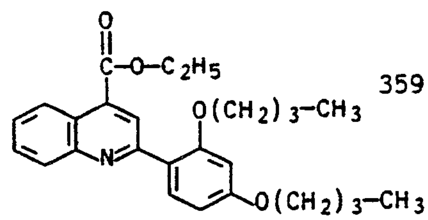 | 359 | 413 | 67 |

Beispiel 38

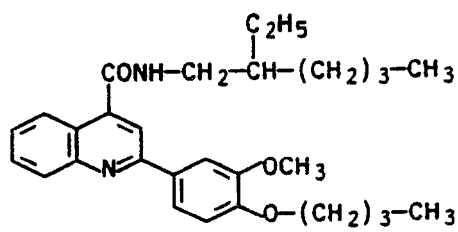

10,5 g 2-(4′-Butoxy-3′-methoxyphenyl)-chinolin-4-carbonsäure (hergestellt analog Beispiel 7) wurde in 50 g Thionylchlorid aufgelöst und das Reaktionsgemisch eine halbe Stunde unter Rückfluß erhitzt. Das überschüssige Thionylchlorid wurde unter Vakuum abdestilliert und der verbleibende Rückstand in 50 g 2-Ethylhexylamin unter Eiswasserkühlung eingetragen. Es wurde 1 Stunde bei 25 °C und eine weitere halbe Stunde bei 80 °C gerührt. Nach dem Abkühlen wurde mit 200 ml Methanol verdünnt und das ausgefallene Produkt mit Methanol und Wasser gewaschen und im Trockenschrank bei 60 °C getrocknet. Es wurden 6,5 g 2-(4′-Butoxy-3′-methoxyphenyl)-chinolin-4-carbonsäure-(2′-ethyl)-hexylamid erhalten. Die farblosen Kristalle schmelzen bei 118 °C und absorbieren in Toluol bei $\lambda_{max}$ 352 nm. In 2 %iger methanolischer Salzsäure absorbiert die Verbindung bei 403 nm.

Beispiel 39

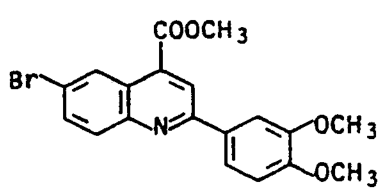

7,68 g 5-Bromisatin, 5,41 g 3,4-Dimethoxyacetophenon, 2 g Tetrabutylammoniumhydrogensulfat und 45

g 33 %ige Kalilauge wurden 10 Stunden unter Rückfluß gerührt. Das ausgefallene Kalisalz wurde abfiltriert, in Wasser suspendiert und mit 10 %iger Salzsäure auf pH 4,5 gestellt. Ausbeute: 9,22 g 6-Brom-2(3′,4′-dimethoxyphenyl)-chinolin-4-carbonsäure. Die Veresterung erfolgte wie in Beispiel 1. Es wurden 6,8 g 6-Brom-2(3′,4′-dimethoxyphenyl)-chinolin-4-carbonsäuremethylester erhalten. Die Substanz schmilzt bei 154 °C und absorbiert in Toluol bei $\lambda_{max}$ 377 nm. In 2 %iger methanolischer Salzsäure absorbiert sie bei $\lambda_{max}$ 425 nm.

Analog Beispiel 39 wurden die in der folgenden Tabelle angegebenen Chinolin-4-carbonsäuremethylester hergestellt.

| Beispiel Nr. | Chinolin-4-carbonsäuremethylester | $\lambda_{max}$ [nm] neutral | sauer |
|---|---|---|---|
| 40 | 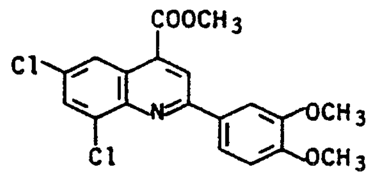 | 385 | 435 |
| 41 | 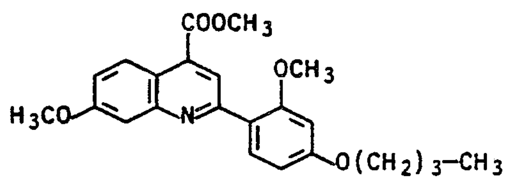 | 366 | 421 |
| 42 | 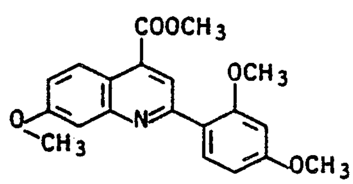 | 363 | 419 |

Die 7-Methoxy-2(2′,4′-dimethoxyphenyl)-chinolin-4-carbonsäure wurde analog Beispiel 7 hergestellt. Die Säure wurde, so wie es für die Beispiele 32 bis 37 angegeben ist, mit den entsprechenden Alkoholen verestert. Die Amidierungen erfolgten nach Beispiel 38.

| Beispiel Nr. | Verbindung | Schmelzpunkt [°C] |
|---|---|---|
| 43 | 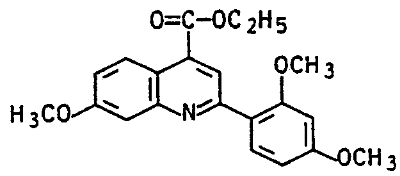 | 97 - 98 |
| 44 | 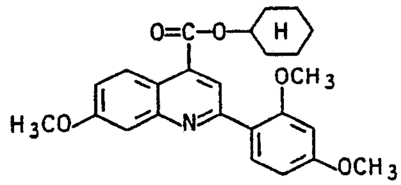 | 83 - 84 |
| 45 | 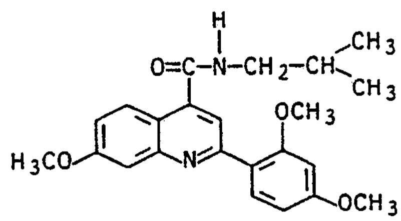 | 129 - 130 |
| 46 | 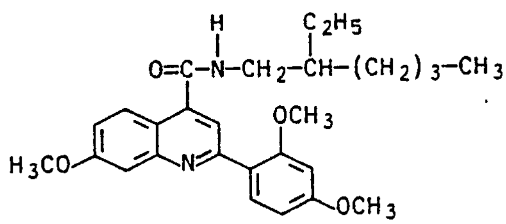 | 104,5 - 105,5 |
| 47 | 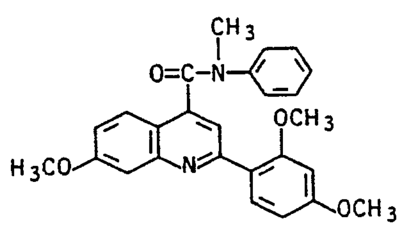 | 185 - 186 |

Analog zu Beispiel 7 können auch die Verbindungen der Beispiele 48 - 52...

| Beispiel Nr. | Verbindung | $\lambda_{max}$ neutral | [nm] sauer |
|---|---|---|---|
| 48 | 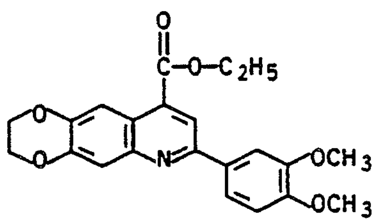 | 373 | 433 |
| 49 | 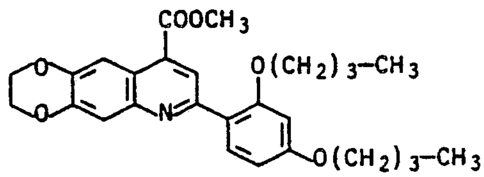 | 369 | 424 |
| 50 | 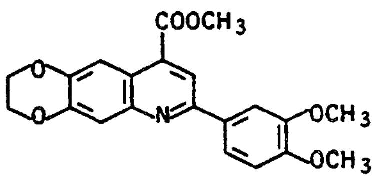 | 373 | 433 |
| 51 | 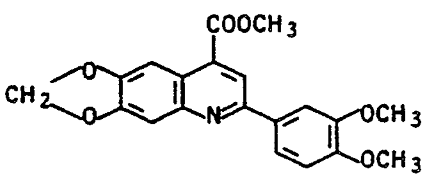 | 370 | 432 |
| 52 | 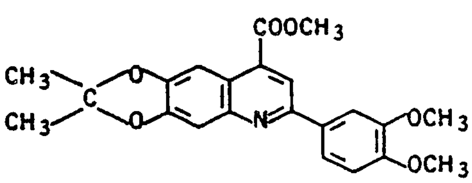 | 371 | 434 |

Beispiel 53

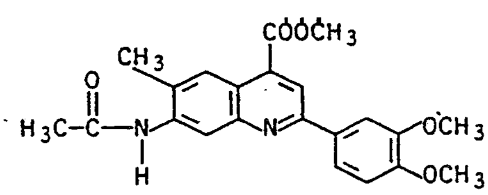

11,4 g (0,03 mol) der analog Beispiel 7 hergestellten 2-(3′,4′-Dimethoxyphenyl)-6-methyl-7-acetamino-chinolin-4-carbonsäure wurde bei Raumtemperatur unter Stickstoffatmosphäre in eine Lösung von 3,8 g (0,03 mol) Oxalylchlorid in 60 ml Toluol eingetragen und unter Eiswasserkühlung mit 6,1 g (0,06 mol) Triethylamin in 30 ml Toluol versetzt. Nach 1,5 Std. bei 65-70 °C wurden 3,86 g (0,03 mol) und nach weiteren 3 Std. 2 g (0,02 mol) Oxalylchlorid hinzugefügt. Das Reaktionsgemisch wurde anschließend 2,5

Std. auf 75-80 °C erhitzt, abgekühlt und mit 30 g Methanol und 10,1 g (0,01 mol) Triethylamin versetzt. Die Aufarbeitung des ausgefallenen Feststoffs erfolgte nach 12-stündigem Rühren bei Raumtemperatur durch Absaugen, Waschen mit Methanol und Wasser und Umkristallisation aus Toluol. Ausbeute: 6,8 g (57 %), Schmelzpunkt: 234-236 °C.

Wie in Beispiel 53 beschrieben, werden die Chinolincarbonsäureester der Beispiele 54 und 55 hergestellt.

| Beispiel Nr. | Chinolin-Derivat | Schmelzpunkt [°C] |
|---|---|---|
| 54 | 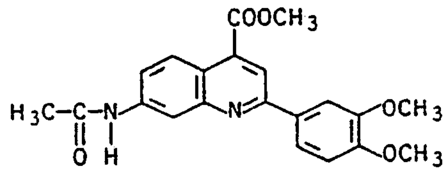 | 255-258 |
| 55 | 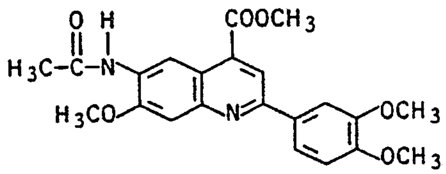 | 248-249 |

Beispiel 56

Der Farbbildner des Beispiels 2 wird 0,5 %ig in einem diisopropylierten Naphthalin gelöst und mit einem 6 µm Rakel auf ein CF-Papier der Fa. Wiggins Teape aufgetragen. Es entwickelt sich ein gelber Farbton. Nach einstündigem Belichten im Suntest-Gerät der Fa. Hanau wird der ΔE-Wert gemäß CIELAB-System ermittelt. Ebenso wird mit den Farbbildnern des Standes der Technik verfahren.

| | ΔE |
|---|---|
| Farbbildner des Beispiels 2 | 3 |
| Farbbildner aus EP-A-109930 Beispiel 2 | 50 |
| Farbbildner aus GB-A-2136823 Beispiel 14 | 14 |
| Farbbildner aus DE-OS-2227597 Beispiel 2 | 40 |

ΔE-Werte von 1 sind deutlich visuell erkennbar.

**Ansprüche**

1. Chinolin-4-carbonsäurederivate der allgemeinen Formel I

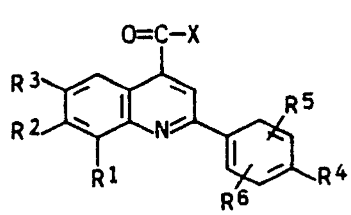

(I)

in der einer der Reste

$R^1$, $R^2$ und $R^3$ für Wasserstoff, die beiden anderen unabhängig voneinander für Wasserstoff, Fluor, Chlor, Brom, lineares und verzweigtes $C_1$-$C_5$ Alkyl, Hydroxy, $C_1$-$C_{10}$ Alkoxy, das im Alkyl durch 1 oder 2 Sauerstoffatome unterbrochen sein kann oder für

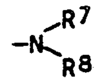

stehen, wobei
$R^7$ Wasserstoff, Methyl oder Ethyl und
$R^8$ $C_1$-$C_4$ Alkanoyl, Benzoyl, p-Chlorbenzoyl, $C_1$-$C_6$ Alkyl, Benzyl oder p-Chlorbenzyl ist, oder
$R^1$ und $R^2$ zusammen einen ankondensierten Benzolring,
$R^2$ und $R^3$ zusammen gegebenenfalls durch $C_1$- bis $C_4$-Alkyl substituierte Methylendioxy oder Ethylendioxy,
$R^4$ Hydroxy, $C_1$-$C_{10}$ Alkoxy, das durch 1 bis 4 Sauerstoffatome unterbrochen sein kann, oder gegebenenfalls durch Chlor substituiertes Phenyl-$C_1$-$C_2$-alkoxy,
$R^5$ Hydroxy oder $C_1$-$C_{10}$ Alkoxy, wobei orthoständiges $R^4$ und $R^5$ zusammen auch Methylendioxy oder 1,2-Ethylendioxy sein können,
$R^6$ Wasserstoff, Hydroxy, Methoxy, Ethoxy, Chlor, Fluor oder $C_1$-$C_4$ Alkyl und
X $C_1$-$C_{20}$ Alkoxy, Benzyloxy, p-Chlorbenzyloxy, Phenylethyloxy, Cyclopentoxy oder Cyclohexoxy oder

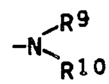

bedeuten, wobei
$R^9$ Wasserstoff, Methyl oder Ethyl und
$R^{10}$ Wasserstoff, $C_1$-$C_{20}$ Alkyl, gegebenenfalls ein- oder zweimal durch Chlor, Methoxy oder Methyl substituiertes Phenyl oder ein Rest der Formel

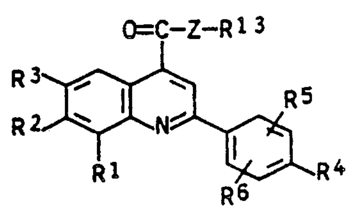

(II) ,

sind, in dem
$R^1$ bis $R^6$ die oben angegebene Bedeutung haben und
Z Sauerstoff oder >N-$R^9$ und
$R^{13}$ lineares oder verzweigtes $C_2$-$C_{10}$-Alkylen, das durch bis zu 4 Sauerstoffatome unterbrochen sein kann, oder 1,2-, 1,3- oder 1,4-Xylylen bedeuten.

2. Chinolin-4-carbonsäurederivate gemäß Anspruch 1, dadurch gekennzeichnet, daß einer der Reste $R^1$, $R^2$ und $R^3$ Wasserstoff, Fluor, Chlor, Brom, lineares oder verzweigtes $C_1$-$C_5$ Alkyl, Hydroxy oder $C_1$-$C_{10}$ Alkoxy, das im Alkyl durch 1 bis 2 Sauerstoffatome unterbrochen sein kann, ist und die restlichen Reste für Wasserstoff stehen.

3. Chinolin-4-carbonsäurederivate gemäß Anspruch 1, bei denen
$R^1$ Wasserstoff und
$R^2$ und $R^3$ gegebenenfalls durch Methyl substituiertes Methylen- oder Ethylendioxy sind.

4. Chinolin-4-carbonsäurederivate gemäß Anspruch 1 oder 2, dadurch gekennzeichnet, daß X für $C_1$-$C_{20}$ Alkoxy, Benzyloxy, Phenylethyloxy, Cyclopentoxy oder Cyclohexoxy oder für

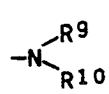

steht, worin

$R^9$ Wasserstoff, Methyl oder Ethyl und
$R^{10}$ $C_1$-$C_{20}$ Alkyl oder gegebenenfalls ein oder zweifach durch Chlor, Methoxy und/oder Methyl substituiertes Phenyl bedeutet.

5. Chinolin-4-carbonsäurederivate gemäß Anspr. 1, gekennzeichnet durch die Formel

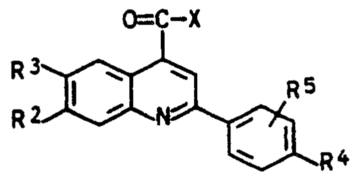

(III) ,

in der
$R^2$, $R^3$, $R^4$ und $R^5$ die im Anspruch 1 angegebene Bedeutung haben und
X für $C_1$-$C_{20}$ Alkoxy oder für

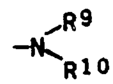

steht, worin
$R^9$ Wasserstoff, Methyl oder Ethyl und
$R^{10}$ $C_1$-$C_{10}$ Alkyl oder Phenyl ist.

6. Chinolin-4-carbonsäurederivate gemäß Anspruch 5, dadurch gekennzeichnet, daß $R^2$ Wasserstoff oder $C_1$-$C_5$ Alkoxy ist.

7. Verwendung der Chinolin-4-carbonsäurederivate gemäß den Ansprüchen 1 bis 6 als Farbbildner in druck- und thermoempfindlichen Aufzeichnungsmaterialien.

Europäisches Patentamt

# EUROPÄISCHER RECHERCHENBERICHT

Nummer der Anmeldung

EP 90 10 3019

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | EP-A-0 256 180 (YAMADA CHEMICAL CO. LTD.)<br>* Seite 16, Anspruch 1, Seite 17, Anspruch 11 *<br>--- | 1,7 | C 07 D 215/52<br>C 07 D 405/04<br>C 07 D 491/056<br>B 41 M 5/136//<br>(C 07 D 491/056<br>C 07 D 317:00<br>C 07 D 215:00 ) |
| A,D | EP-A-0 159 295 (CIBA-GEIGY)<br>* Seite 27, Anspruch 1, Seite 33, Anspruch 18 *<br>--- | 1,7 | |
| A | DE-C- 244 788 (CHEMISCHE FABRIK AUF AKTIEN IN BERLIN)<br>* gesamtes Dokument *<br>--- | 1 | |
| A | EP-A-0 133 244 (E.I. DU PONT DE NEMOURS & CO.)<br>* Seite 62, Anspruch 28 *<br>----- | 1 | |
| | | | **RECHERCHIERTE SACHGEBIETE (Int. Cl.5)** |
| | | | C 07 D 215/00<br>C 07 D 405/00<br>C 07 D 491/00<br>B 41 M 5/00 |

Der vorliegende Recherchenbericht wurde für alle Patentansprüche erstellt

| Recherchenort | Abschlußdatum der Recherche | Prüfer |
|---|---|---|
| BERLIN | 10-05-1990 | KYRIAKAKOU G |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)